# EUROPEAN PATENT APPLICATION

(11) **EP 1 142 562 A1**
(43) Date of publication of application: **10.10.2001**
(21) Application number: 99959948.3
(22) Date of filing: 21.12.1999
(51) Int. Cl.: A61K 7/13

(54) **HAIR DYE COMPOSITIONS**

(30) Priority: 22.12.1998 JP 36420298
(71) Applicant: Lion Corporation, Tokyo 130-8644 (JP)
(72) Inventor: ONUKI, Takeshi Lion Corporation, Tokyo 130-8644 (JP); NOGUCHI, Mutsumi Lion Corporation, Tokyo 130-8644 (JP); MITAMURA, Joji Lion Corporation, Tokyo 130-8644 (JP)
(74) Representative: Stuart, Ian Alexander
(86) International application number: JP9907188
(87) International publication number: WO0037031

(57) **Abstract**

A laccase-containing oxidation hair dye composition of one-pack type in the form of cream or gel which is packed into a container with a good oxygen barrier property.

## Description

### TECHNICAL FIELD

The present invention relates to an oxidation hair dye composition of one-pack type which is incorporated with laccase. More particularly, the present invention relates to an oxidation hair dye composition of one-pack type which keeps laccase stable for a long time and produces a good hair-dyeing effect.

### BACKGROUND ART

An ordinary oxidation hair dye is composed of a dye precursor (primary ingredient) and an oxidizing agent (secondary ingredient) which are mixed together for reaction before use and the resulting mixture is applied to white hair to be dyed.

The dye precursor as the primary ingredient is p-phenylenediamine, p-aminophenol, or the like. It is usually alkaline and hence it poses a problem with irritant action on the scalp.

The oxidizing agent as the secondary ingredient is usually hydrogen peroxide. It is known that hydrogen peroxide damages hair and damaged hair turns reddish-brown after continued use for a long time.

An attempt has been made to tackle the first problem by making the primary ingredient neutral (in Japanese Patent Laid-open No. Hei 8-217652). Another attempt has been made to tackle the second problem by replacing hydrogen peroxide with an oxidase. Examples of oxidases include peroxidase (Japanese Patent Laid-open Nos. Sho 47-10400 and Sho 53-32132), laccase (US Patent No. 3251742 and Japanese Patent Laid-open No. Hei 6-172145), and uricase (Japanese Patent Laid-open No. Sho 63-246313). These disclosed technologies still have disadvantages. That is, peroxidase needs hydrogen peroxide to be added to the hair dye system because of the inherent characteristics of enzyme. Also, uricase utilizes hydrogen peroxide evolved by the enzymatic reaction; therefore, it does not eliminate troubles due to hydrogen peroxide.

By contrast, laccase damages hair less than peroxidase and uricase because it does not need the hair dye system to contain hydrogen peroxide. The disadvantage of laccase used as an ingredient of a hair dye composition of one-pack type is that it reacts with the dye precursor in the composition, giving rise to insoluble aggregates, during storage, because it is an unstable protein, so that laccase cannot fully produce its effect. This is a serious problem to be solved before laccase is adopted for commercial hair dye compositions of one-pack type.

There have been disclosed technologies to improve the storage stability of oxidase such as catalase and uricase in Japanese Patent Laid-open Nos. Hei 8-175935 and Hei 8-217652, respectively. However, there is no known technology to improve the stability of laccase.

### DISCLOSURE OF INVENTION

It is an object of the present invention to provide a laccase-containing oxidation hair dye composition of one-pack type which keeps laccase stable, without forming aggregates, and permits laccase to fully produce its effect.

In order to achieve the above-mentioned object, the present inventors carried out extensive studies and completed the present invention.
(1) The first aspect of the present invention resides in a laccase-containing oxidation hair dye composition of one-pack type in the form of cream or gel which is packed into a container with a good oxygen barrier property.
(2) The second aspect of the present invention resides in a laccase-containing oxidation hair dye composition of one-pack type which is packed into a container having an oxygen absorber in its head space.

Despite its one-pack type, the laccase-containing oxidation hair dye composition according to the present invention keeps the laccase very stable for a long time, significantly preventing reaction with the dye precursor to form aggregates, and produces a good hair dyeing effect.

### BEST MODE FOR CARRYING OUT THE INVENTION

A detailed description of the invention is given below. According to the present invention, the hair dye composition contains laccase as an oxidizing agent. The laccase used in the present invention is an enzyme designated as E.C.1.10.3.2. Laccase oxidizes urushiol in the sap of lacquer tree, thereby forming Japanese lacquer. Laccase is also ubiquitous in many plants and microorganisms, and it catalyzes the oxidation of aromatic compounds. The origin of laccase is not restricted in the present invention.

The hair dye composition of the present invention may be incorporated with laccase in an amount of 0.0005 to 30% (by weight), preferably 0.005 to 20%, depending on the form of composition, the frequency of use, the time required for processing, and the enzyme potency. With an amount less than 0.0005%, laccase does not fully produce its effect. With an amount more than 30%, laccase does not produce any additional effect in proportion to the amount.

The hair dye composition of the present invention contains an dye precursor which is involved in color reaction with laccase. The dye precursor in the present invention is not specifically restricted in its kind and amount. It may be chosen from any known ones. It includes the following examples, which are listed in the standards of raw materials for quasi drugs: 5-amino-o-cresol, o-aminophenol, m-aminophenol, p-aminophenol, 2,6-diaminopyridine, 5-(2-hydroxyethylamino)-2-methylphenol, N,N-bis(β-hydroxyl)-p-phenylenediamine sulfate, p-nitro-o-phenylenediamine, p-phenylenediamine, m-phenylenediamine, N-phenyl-p-phenylenediamine, resorcinol, 2-hydroxy-5-nitro-2',4'-diaminobenzene sodium sulfate, toluene-2,5-diamine, 5-amino-o-cresol sulfate, p-aminophenol sulfate, o-chloro-p-phenylenediamine sulfate, 4,4'-diaminodiphenylamine sulfate, p-methylaminophenol sulfate, p-phenylenediamine sulfate, m-phenylenediamine sulfate, toluene-2,5-diamine sulfate, 2,4-diaminophenoxyethanol hydrochloride, toluene-2,5-diamine hydrochloride, m-phenylenediamine hydrochloride, 2,4-diaminophenol hydrochloride, 3,3'-iminodiphenol, p-phenylenediamine hydrochloride, N-phenyl-p-phenylenediamine hydrochloride, N-phenyl-p-phenylenediamine acetate, 1,5-dihydroxynaphthalene, toluene-3,4-diamine, p-methylaminophenol, N,N'-bis(4-aminophenyl)-2,5-diamino-1,4-quinonediimine, o-aminophenol sulfate, 2,4-diaminophenol sulfate, and m-aminophenol sulfate. They may be used in an adequate amount alone or in combination with one another.

The above-mentioned dye precursor may be used in combination with any of the following direct materials: 2-amino-4-nitrophenol, 2-amino-5-nitrophenol, 1-amino-4-methylaminoanthraquinone, nitro-p-phenylenediamine hydrochloride, 1,4-diaminoanthraquinone, nitro-p-phenylenediamine, picramic acid, sodium picramate, 2-amino-5-nitrophenol sulfate, resorcinol, nitro-p-phenylenediamine sulfate, p-nitro-o-phenylenediamine sulfate, and p-nitro-m-phenylenediamine sulfate.

Of the above-mentioned dye precursors, the following are particularly desirable: p-phenylenediamine or a salt thereof, toluene-2,5-diamine or a salt thereof, p-aminophenol, 5-amino-o-cresol, m-aminophenol, p-nitro-o-phenylenediamine, 2,6-diaminopyridine, resorcinol, o-aminophenol, and m-phenylenediamine.

A substance like a melanine precursor represented by the general formula (1) below is also used to meet the recent nature-oriented taste.

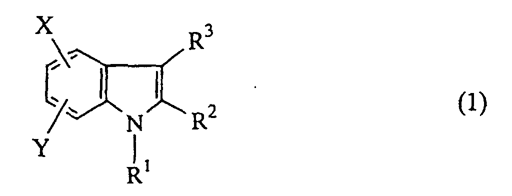

In the general formula (1), X denotes a hydrogen atom, NH₂, OH, or any of linear or branched alkyl group having 1 to 6 carbon atoms, alkenyl group, and alkoxyl group; and Y denotes OH or NH₂. If X is OH or any of linear or branched alkyl group having 1 to 6 carbon atoms, alkenyl group, and alkoxyl group, X is at the 5-, 6-, or 7-position of the ring and the ortho position with respect to Y.

R¹ and R³ are identical or different, each denoting a hydrogen atom or any of linear or branched alkyl group having 1 to 6 carbon atoms, alkenyl group, and alkoxyl group; and R² denotes any of linear or branched alkyl group having 1 to 6 carbon atoms, alkenyl group, alkoxyl group, and carboxyl group.

The compound represented by the general formula (1) above includes the following: 4,5-dihydroxyindole, 5,6-dihydroxyindole, 6,7-dihydroxyindole, N-methyl-5,6-dihydroxyindole, N-ethyl-5,6-dihydroxylindole, N-hexyl-5,6-dihydroxyindole, 2-methyl-5,6-dihydroxyindole, 3-methyl-5,6-dihydrxoxyindole, 4-hydroxyindole, 2,3-dimethyl-5,6-dihydroxyindole, 2-methyl-5-ethyl-6-hydroxyindole, 2-methyl-5-hydroxy-6-β-hydroxyethylindole, 4-hydroxypropylindole, 2-hydroxy-3-methoxyindole, 4-hydroxy-5-methoxyindole, 6-hydroxy-7-methoxyindole, 6-hydroxy-5-methoxyindole, 6-hydroxyindole, 5-hydroxyindole, 7-hydroxyindole, 7-aminoindole, 5-aminoindole, 4-aminoindole, 5,6-dihydroxyindole carboxylic acid, and 1-methyl-5,6-dihydroxyindole and a salt thereof.

A substance like a melanine precursor represented by the general formula (2) below is also desirable.

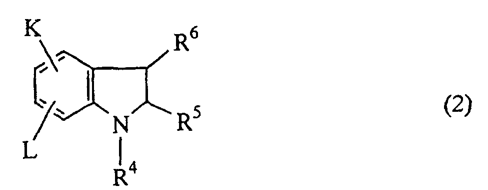

In the general formula (2), K denotes a hydrogen atom, NH₂, OH, or any of linear or branched alkyl group having 1 to 6 carbon atoms, alkenyl group, and alkoxyl group; and L denotes OH or NH₂. If K is OH or any of linear or branched alkyl group having 1 to 6 carbon atoms, alkenyl group, and alkoxyl group, K is at the 5-, 6-, or 7-position of the ring and the ortho position with respect to L.

R⁴ and R⁶ are identical or different, each denoting a hydrogen atom or any of linear or branched alkyl group having 1 to 6 carbon atoms, alkenyl group, and alkoxyl group; and R⁵ denotes any of linear or branched alkyl group having 1 to 6 carbon atoms, alkenyl group, alkoxyl group, and carboxyl group.

The compound represented by the general formula (2) above includes the following: 4,5-dihydroxyindoline, 5,6-dihydroxyindoline, 6,7-dihydroxyindoline, N-methyl-5,6-dihydroxyindoline, N-ethyl-5,6-dihydroxylindoline, N-hexyl-5,6-dihydroxyindoline, 2-methyl-5,6-dihydroxyindoline, 3-methyl-5,6-dihydrxoxyindoline, 4-hydroxyindoline, 2,3-dimethyl-5,6-dihydroxyindoline, 2-methyl-5-ethyl-6-hydroxyindoline, 2-methyl-5-hydroxy-6-β-hydroxyethylindoline, 4-hydroxypropylindoline, 2-hydroxy-3-methoxyindoline, 4-hydroxy-5-methoxyindoline, 6-hydroxy-7-methoxyindoline, 6-hydroxy-5-methoxyindoline, 6-hydroxyindoline, 5-hydroxyindoline, 7-hydroxyindoline, 7-aminoindoline, 5-aminoindoline, 4-aminoindoline, 5,6-dihydroxyindoline carboxylic acid, and 1-methyl-5,6-dihydroxyindoline and a salt thereof.

The above-mentioned dye precursors may be used alone or in combination with one another according to the color desired.

The content of the dye precursor may be properly established according to the frequency of use and the form of composition. Usually, it is 0.01-10%, preferably 0.1-5%, of the total amount of the composition.

The composition may contain, in addition to the above-mentioned ingredients, such optional ingredients as pH buffer, surface active agent, thickener (e.g., hydroxyethylcellulose and xanthan gum), perfume, preservative, UV light absorber, antioxidant, disinfectant, and pearlescent pigment. The surface active agent includes anionic surface active agents (such as α-olefinsulfonate, alkanesulfonate, fatty acid alkyl ether carboxylate, salt of N-acylamino acid, and C₁₂-C₁₈ saturated or unsaturated fatty acid acyl glutamic acid ester), amphoteric surface active agents (such as alkylbetaine, alkylamidebetaine, and hydroxysulfobetaine), cationic surface active agents (such as mono- or dialkyl quaternary ammonium salt), and nonionic surface active agents (such as polyoxyethylene alkyl ether and fatty acid alkylolamide).

The composition may be incorporated with silicone derivatives (such as dimethylpolysiloxane, amino-modified silicone, and polyether-modified silicone) for improvement in hair touch.

The composition should have pH 5.0-9.0, preferably pH 6.0-8.0. An excessively high pH causes skin irritation.

According to the first aspect of the present invention, the above-mentioned ingredients (such as laccase and dye precursor) are made into a composition in the form of cream or gel, and the resulting composition is packed into a container having a good oxygen barrier property. The hair dye composition of one pack-type obtained in this way remains stable and keeps laccase stable for a long time and produces a good hair-dyeing effect.

The container with a good oxygen barrier property may be made from polyester, ethylene-vinyl alcohol copolymer, polyvinylidene chloride, and polyacrylonitrile, which effectively prevent oxygen permeation. It may also be made from such general-purpose resin as polyethylene and polypropylene having a poor oxygen barrier property if they are laminated or coated with a resin having a good oxygen barrier property. A good oxygen barrier property is also obtained by vapor deposition with aluminum or silica or incorporation with a radical inhibitor. The container should have an oxygen permeability of 0.01-10 cc/24 hr · atm (25°C, dry). The structure of the container is not specifically restricted; however, it should preferably be a tube.

The first aspect of the present invention should preferably be embodied in such a way that the mixing of the above-mentioned ingredients is carried out in the absence of oxygen, the concentration of oxygen in the composition is equal to or less than 0.00015%, the packing of the composition is carried out in the absence of oxygen, and the oxygen concentration in the head space of the container is equal to or less than 1% or the container has an oxygen absorber in its head space. The container with a good oxygen barrier property and the mixing of ingredients in an oxygen-free atmosphere prevent the enzyme from catalyzing the reaction of the dye precursor with oxygen in the head space of the container. Therefore, the resulting composition (product) remains very stable without causing the dye precursor to form aggregates.

According to the second aspect of the present invention, the container into which the composition is packed is provided with an oxygen absorber in its head space. In this case, the oxygen concentration in the space head is not specifically restricted in the initial state. It may be equal to that in the case of ordinary packing. However, the oxygen concentration in the head space should preferably be equal to or less than 1%, particularly equal to or less than 0.1%.

The oxygen absorber used in the second aspect of the present invention is not specifically restricted so long as it absorbs oxygen in the head space of the container. It may be an iron-based one or an organic one, such as "Ageless SA20" commercially available from Mitsubishi Gas Chemical Company, Inc. The oxygen absorber prevents the enzyme from catalyzing the reaction of the dye precursor with oxygen in the head space of the container during mixing. Therefore, the atmosphere in the head space becomes free of oxygen and the composition (product) in the container remains very stable without causing the dye precursor to form aggregates. The composition packed in a tube remains stable during its continued use because the oxygen absorber absorbs the oxygen which has entered the head space each time the tube is opened. The amount of the oxygen absorber should be such that the oxygen concentration in the head space is kept equal to or less than 1%.

As mentioned above, the present invention is directed to a permanent hair dye composition of one-pack type, which is ready to use requiring no mixing prior to use unlike the conventional one of two-pack type and hence is convenient for consumers. The hair dye composition is available in the form of aerosol, cream, gel, or liquid, and remains very stable without the dye precursor therein forming aggregates.

In what follows, the invention will be described with reference to examples and comparative examples which are not intended to restrict the scope thereof. (In examples, "%" means "% by weight".)

Samples in the examples were evaluated in the following manner.

### Presence of aggregates:

After preparation, compositions were allowed to stand for one month at 25°C and 45°C and then visually examined for the presence or absence of aggregates.
- ⓞ :: none
- ○ :: a few
- Δ :: some
- × :: many

### Hair-dyeing ability (ΔE):

A wisp of dry goat white hair (about 10 g) is shampooed. After dewatering, the wet hair (17 g) is rapidly treated with a freshly prepared sample of the composition (3 g each). The treated hair is allowed to stand at 30°C for a prescribed period of time. Finally, the dyed hair is rinsed, dried, shampooed, and air dried. The thus obtained hair sample is tested for the L value, a value, and b value by using a chroma meter (Model SE2000 made by Nippon Denshokusha) The hair-dyeing ability is rated according to the color difference (ΔE) from undyed hair. The greater the value of ΔE, the better the hair-dyeing ability.

### Color migration:

A wisp of human black hair (about 10 g) is dyed with a freshly prepared sample of the composition. The dyed hair is shampooed and dried with a white towel. The towel is visually examined for migrated color.
- ⓞ :: none
- ○ :: slightly noticeable
- Δ :: apparently noticeable
- × :: severely colored

### [Examples 1 to 3 and Comparative Examples 1 and 2]

Hair dye compositions were prepared according to the following formulations. They were packed into those tubes which are constructed as shown in Table 1. The filled tubes were stored for one month at 25° C or 45° C. After storage, the samples were evaluated.

### Example 1 and Comparative Example 1 (in gel form)

| | |
|---|---|
| p-Phenylenediamine | 1 % |
| p-Aminophenol | 0.1 |
| m-Aminophenol | 0.2 |
| Laccase | 1 |
| Xanthan gum | 0.5 |
| Hydroxyethylcellulose | 0.5 |
| Monoethanolamine | suitable amount |
| Purified water | Balance |
| Total | 100.0% |
| pH : 7 (adjusted with monoethanolamine) | |

### Example 2 and Comparative Example 2 (in cream form)

| | |
|---|---|
| p-Phenylenediamine | 0.5 % |
| Toluene-2,5-diamine sulfate | 1 |
| Hydroxypropyl-β-cyclodextrin | 20 |
| Decaglyceryl monostearate | 3 |
| Cetostearyl alcohol | 0.5 |
| Stearic acid | 0.8 |
| Xanthan gum | 1 |
| Laccase | 3 |
| Carboxymethylcellulose | 1 |
| Sodium hydroxide | suitable amount |
| Purified water | Balance |
| Total | 100.0% |
| pH : 7 (adjusted with sodium hydroxide) | |

### Example 3 (in cream form)

| | |
|---|---|
| p-Phenylenediamine | 2 % |
| Toluene-2,5-diamine sulfate | 1 |
| m-Phenylenediamine | 0.1 |
| m-Aminophenol | 0.8 |
| Hydroxypropyl-β-cyclodextrin | 45 |
| POE (10) cetyl ether | 8 |
| Stearyl alcohol | 2.5 |
| Oleyl alcohol | 5 |
| Behenyl alcohol | 2 |
| Cetyl alcohol | 2 |
| Stearyltrimethylammonium chloride | 1 |
| Laccase | 3 |
| Glycerin | 2 |
| Triethanolamine | suitable amount |
| Purified water | Balance |
| Total | 100.0% |
| pH : 7 (adjusted with triethanolamine) | |

**Table 1**

| | | Example | | | Comparative Example | |
|---|---|---|---|---|---|---|
| | | 1 | 2 | 3 | 1 | 2 |
| Construction of tube | | *LDPE*/ethylene vinyl alcohol copolymer/LDPE | *LDPE*/ethylene vinyl alcohol copolymer/PP | Aluminum-deposited PET /ethylene vinyl alcohol copolymer/LDPE | LDPE/PP | LDPE/PP /LDPE |
| Aggregates | One month, at 25°C | ⓞ | ⓞ | ⓞ | Δ ∼ × | × |
| | One month, at 45°C | ⓞ | ⓞ | ⓞ | × | × |
| Hair dyeing ability (ΔE), after storage for one month, at 45°C | | 29 | 32 | 30 | 25 | 26 |
| Color migration, after storage for one month, at 45°C | | ⓞ | ⓞ | ⓞ | ○ | ○ |
| LDPE : Low-density polyethylene | | | | | | |
| PET : Polyethylene terephthalate | | | | | | |
| PP : Polypropylene | | | | | | |

### [Examples 4 to 7 and Comparative Example 3 to 6]

Hair dye compositions (in aerosol form) were prepared in the usual way according to the formulations shown in Table 2. They were packed into aerosol containers which contain 1% oxygen in the head space. In Examples, the aerosol containers are provided with an iron-based oxygen absorber ("Ageless SA20", capable of absorbing 20 ml of oxygen). The results of evaluation are shown in Table 2.

**Table 2**

| | | Example | | | | Comparative Example | | | |
|---|---|---|---|---|---|---|---|---|---|
| | | 4 | 5 | 6 | 7 | 3 | 4 | 5 | 6 |
| p-Phenylenediamine | | 1.5 | 1.5 | - | 1 | 1.5 | 1.5 | - | 1 |
| p-Aminophenol | | 0.1 | 0.1 | 0.1 | 0.1 | 0.1 | 0.1 | 0.1 | 0.1 |
| m-Aminophenol | | - | - | 0.08 | 0.08 | - | - | 0.08 | 0.08 |
| Toluene-2,5-diamine sulfate | | - | - | 2 | 2 | - | - | 2 | 2 |
| m-Phenylenediamine | | 0.15 | 0.15 | - | - | 0.15 | 0.15 | - | - |
| Resorcinol | | - | - | - | 0.1 | - | - | - | 0.1 |
| Laccase | | 1 | 5 | 3 | 10 | 1 | 5 | 3 | 10 |
| Monoethanolamine | | s.a. | s.a. | s.a. | s.a. | s.a. | s.a. | s.a. | s.a. |
| Purified water | | balance | balance | balance | balance | balance | balance | balance | balance |
| LPG (4.0 kg/cm²) | | 4 | 4 | 4 | 4 | 4 | 4 | 4 | 4 |
| Total (%) | | 100.0 | 100.0 | 100.0 | 100.0 | 100.0 | 100.0 | 100.0 | 100.0 |
| pH (adjusted with monoethanolamine) | | 7.0 | 7.0 | 7.0 | 7.0 | 7.0 | 7.0 | 7.0 | 7.0 |
| Iron-based oxygen absorber (capable of absorbing 20 ml of oxygen) | | yes | yes | yes | yes | no | no | no | no |
| Aggregates | One month, at 25°C | ⓞ | ⓞ | ⓞ | ⓞ | Δ~× | × | Δ~× | × |
| | One month, at 45°C | ⓞ | ⓞ | ⓞ | ⓞ | × | × | × | × |
| Hair-dyeing ability (ΔE), after storage for one month, at 45°C | | 29 | 32 | 30 | 34 | 23 | 25 | 24 | 27 |
| Color migration, after storage for one month at 45°C | | ⓞ | ⓞ | ⓞ | ⓞ | ○ | ○ | Δ | Δ |

| | | | | | | | | | |
|---|---|---|---|---|---|---|---|---|---|
| s.a.: suitable amount | | | | | | | | | |

### [Example 8] (in gel form)

| | |
|---|---|
| p-Phenylenediamine | 1 % |
| p-Aminophenol | 0.1 |
| m-Aminophenol | 0.2 |
| Laccase | 1 |
| Xanthan gum | 0.5 |
| Hydroxyethylcellulose | 0.5 |
| Monoethanolamine | suitable amount |
| Purified water | Balance |
| Total | 100.0% |
| pH : 7 (adjusted with sodium hydroxide) | |
| Provided with oxygen absorber (capable of absorbing 20 ml of oxygen) | |

### [Example 9] (in cream form)

| | |
|---|---|
| p-Phenylenediamine | 0.5 % |
| Toluene-2,5-diamine sulfate | 1 |
| Hydroxypropyl-β-cyclodextrin | 20 |
| Decaglyceryl monostearate | 3 |
| Cetostearyl alcohol | 0.5 |
| Stearic acid | 0.8 |
| Xanthan gum | 1 |
| Laccase | 0.1 |
| Carboxymethylcellulose | 1 |
| Monoethanolamine | suitable amount |
| Purified water | Balance |
| Total | 100.0% |
| pH : 7 (adjusted with monoethanolamine) | |
| Provided with oxygen absorber (capable of absorbing 20 ml of oxygen) | |

### [Example 10] (in cream form)

| | |
|---|---|
| p-Phenylenediamine | 2 % |
| Toluene-2,5-diamine sulfate | 1 |
| m-Phenylenediamine | 0.1 |
| m-Aminophenol | 0.8 |
| Hydroxypropyl-β-cyclodextrin | 45 |
| POE (10) cetyl ether | 8 |
| Stearyl alcohol | 2.5 |
| Oleyl alcohol | 5 |
| Behenyl alcohol | 2 |
| Cetyl alcohol | 2 |
| Stearyltrimethylammonium chloride | 1 |
| Laccase | 3 |
| Glycerin | 2 |
| Triethanolamine | suitable amount |
| Purified water | Balance |
| Total | 100.0% |
| pH : 7 (adjusted with triethanolamine) | |
| Provided with oxygen absorber (capable of absorbing 20 ml of oxygen) | |

The composition (in gel form) in Example 8 and the compositions (in cream form) in Examples 9 and 10 were packed into aluminum tubes, and the resulting products were evaluated in the same way as in Examples 4 to 7. They passed all the tests and exhibited good storage stability, hair dyeing ability, and non-color migration.

### [Example 11] (Permanent hair dye composition of one-pack type, in foam form)

| (Stock solution) | |
|---|---|
| p-Phenylenediamine | 1.0 % |
| 2,5-Diaminotoluene sulfate | 2.0 |
| m-Phenylenediamine | 0.5 |
| p-Aminophenol | 0.5 |
| 2-(2'-Hydroxyethylamino)-5-aminotoluene sulfate | 0.15 |
| Oleic acid | 0.2 |
| Oleyl alcohol | 0.2 |
| β-Cyclodextrin | 1.0 |
| Laccase | 1.0 |
| Acetylcysteine | 1.0 |
| Xanthan gum | 0.5 |
| Sorbitan monolaurate | 1.0 |
| Ethanol | 10.0 |
| Glycolic acid | 0.2 |
| Purified water | Balance |
| Total | 100.0% |
| pH : 7.5 (adjusted with monoethanolamine) | |
| Provided with oxygen absorber (capable of absorbing 20 ml of oxygen) | |

The stock solution prepared as mentioned above was packed into an aerosol can. After clinching in a vacuum, the aerosol can was filled with LPG (as a propellant at 2.0 kg) in such an amount that the ratio of stock solution to propellant is 95 : 5 (by weight). Thus there was obtained the desired hair dye composition in aerosol form.

The LPG as a propellant may be replaced by any of nitrogen, carbon dioxide gas, dinitrogen oxide gas, CFCs 11, 12 and 114. They may be used alone or in combination with one another. The aerosol may be of direct spray type (packed in aluminum cans or tin cans) or of dual-container type (piston type, back-in type, or EXXEL type).

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in blue black. This color was the same as that obtained with the fresh composition.

### [Example 12] (Permanent hair dye composition of one-pack type, in foam form)

| (Stock solution) | |
|---|---|
| 5,6-Dihydroxyindoline hydrobromide | 1.0 % |
| 5,6-Dihydroxyindole hydrochloride | 1.0 |
| N-Ethyl-5,6-dihydroxyindole hydrochloride | 0.05 |
| Linoleic acid | 0.2 |
| Oleyl alcohol | 0.2 |
| β-cyclodextrin | 1.0 |
| Laccase | 3.0 |
| Acetylcysteine | 1.0 |
| Hydroxyethylcellulose | 0.5 |
| Sodium coconut oil fatty acid acyl-glutamate | 1.0 |
| Ethanol | 10.0 |
| Lactic acid | 0.2 |
| Purified water | Balance |
| Total | 100.0% |
| pH : 7.5 (adjusted with monoethanolamine) | |
| Provided with oxygen absorber (capable of absorbing 20 ml of oxygen) | |

The stock solution prepared as mentioned above was packed into an aerosol can. After clinching in a vacuum, the aerosol can was filled with LPG (as a propellant at 2.0 kg) in such an amount that the ratio of stock solution to propellant is 95 : 5 (by weight). Thus there was obtained the desired hair dye composition in aerosol form.

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in black. This color was the same as that obtained with the fresh composition.

### [Example 13] (in cream form)

| | |
|---|---|
| 5,6-Dihydroxyindoline hydrobromide | 1.0 % |
| 5,6-Dihydroxyindole hydrochloride | 1.0 |
| N-Methyl-5,6-dihydroxyindline hydrobromide | 0.05 |
| N-Methyl-5,6-dihydroxyindole hydrochloride | 0.05 |
| 2,5-Diaminotoluene sulfate | 0.01 |
| β-Cyclodextrin | 1.0 |
| Laccase | 10.0 |
| Alkyltrimethylammonium chloride | 0.5 |
| Coconut oil fatty acid acyl-L-arginine | 0.5 |
| ethyl-D,L-pyrrolidone carboxylate | |
| Cetostearyl alcohol | 2.0 |
| Oleyl alcohol | 1.0 |
| POE (40) hydrogenated castor oil | 0.75 |
| POE (20) stearyl ether | 0.75 |
| Sorbitan sesquistearate | 1.0 |
| Methyl p-hydroxybenzoate | 0.3 |
| Propylene glycol | 5.0 |
| Glycolic acid | 0.2 |
| Purified water | Balance |
| Total | 100.0% |
| pH : 7.5 (adjusted with monoethanolamine) | |
| Provided with oxygen absorber (capable of absorbing 20 ml of oxygen) | |

The hair dye composition prepared as mentioned above was packed into a tube in the same way as in Example 3. The tube was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in dark brown. This color was the same as that obtained with the fresh composition.

### [Example 14] (in cream form)

| | |
|---|---|
| 2,5-Diaminotoluene sulfate | 2.0 % |
| 2,6-Diaminopyridine | 0.05 |
| N,N-Bis(β-hydroxyl)-p-phenylenediamine | 0.1 |
| 2-Amino-5-o-phenol | 0.5 |
| 2-(2'-Hydroxyethylamino)-5-aminotoluene | 0.15 |
| Linoleic acid | 0.2 |
| β-Cyclodextrin | 1.0 |
| Laccase | 2.0 |
| Stearyltrimethylammonium chloride | 0.5 |
| Behenyltrimethylammonium chloride | 0.5 |
| Cetostearyl alcohol | 2.0 |
| Oleyl alcohol | 1.0 |
| POE (40) glycerin triisostearate | 0.75 |
| POE (20) lauryl ether | 0.75 |
| Sorbitan monostearate | 1.0 |
| Methyl p-hydroxybenzoate | 0.3 |
| Propylene glycol | 5.0 |
| Lactic acid | 0.2 |
| Purified water | Balance |
| Total | 100.0% |
| pH : 7.5 (adjusted with monoethanolamine) | |
| Provided with oxygen absorber (capable of absorbing 20 ml of oxygen) | |

The hair dye composition prepared as mentioned above was packed into a tube in the same way as in Example 2. The tube was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in bright brown. This color was the same as that obtained with the fresh composition.

### [Example 15] (Permanent hair dye composition of one-pack type, in treatment form)

| | |
|---|---|
| 2,5-Diaminotoluene sulfate | 5.0 % |
| 2-Amino-4-nitrophenol | 3.0 |
| 5-Amino-o-cresol | 1.0 |
| p-Aminophenol | 1.0 |
| Oleic acid | 0.5 |
| Linoleic acid | 0.5 |
| β-Cyclodextrin | 2.0 |
| Laccase | 3.0 |
| Stearyltrimethylammonium chloride | 0.5 |
| Cetyltrimethylammonium chloride | 0.5 |
| Cetostearyl alcohol | 4.0 |
| Oleyl alcohol | 1.0 |
| Ethyl oleate | 0.5 |
| Isopropyl palmitate | 0.5 |
| Liquid paraffin | 1.0 |
| Bees wax | 0.5 |
| POE (40) hydrogenated castor oil triisostearate | 0.25 |
| POE (20) hydrogenated castor oil triisostearate | 0.25 |
| POE (30) stearyl ether | 0.75 |
| Sorbitan monostearate | 1.0 |
| Glycerin monostearate | 0.5 |
| Methyl p-hydroxybenzoate | 0.3 |
| Propylene glycol | 5.0 |
| Glycolic acid | 0.2 |
| Purified water | Balance |
| Total | 100.0% |
| pH : 7.5 (adjusted with monoethanolamine) | |
| Provided with oxygen absorber (capable of absorbing 20 ml of oxygen) | |

The hair dye composition prepared as mentioned above was packed into a tube in the same way as in Example 2. The tube was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in reddish brown. This color was the same as that obtained with the fresh composition. The hair treated with this hair dye composition gave a good touch due to treatment effect.

### [Example 16] (Permanent hair dye composition of one-pack type, in treatment form)

| | |
|---|---|
| 5,6-Dihydroxyindoline hydrobromide | 1.0 % |
| 5,6-Dihydroxyindole hydrochloride | 1.0 |
| N-Methyl-5,6-dihydroxyindoline hydrobromide | 0.5 |
| N-Methyl-5,6-dihydroxyindole hydrochloride | 0.5 |
| 5-Aminoindole hydrochloride | 0.25 |
| 2,3-Dimethyl-5,6-dihydroxyindoline hydrobromide | 0.25 |
| Oleic acid | 0.5 |
| Linoleic acid | 0.5 |
| β-Cyclodextrin | 2.0 |
| Laccase | 5.0 |
| Stearyltrimethylammonium chloride | 0.5 |
| Cetyltrimethylammonium chloride | 0.5 |
| Cetostearyl alcohol | 4.0 |
| Oleyl alcohol | 1.0 |
| Ethyl oleate | 0.5 |
| Isopropyl palmitate | 0.5 |
| Liquid paraffin | 1.0 |
| Bees wax | 0.5 |
| POE (40) hydrogenated castor oil triisostearate | 0.25 |
| POE (20) hydrogenated castor oil triisostearate | 0.25 |
| POE (30) stearyl ether | 0.75 |
| Sorbitan monostearate | 1.0 |
| Glycerin monostearate | 0.5 |
| Methyl p-hydroxybenzoate | 0.3 |
| Propylene glycol | 5.0 |
| Lactic acid | 0.2 |
| Purified water | Balance |
| Total | 100.0% |
| pH : 7.5 (adjusted with monoethanolamine) | |
| Provided with oxygen absorber (capable of absorbing 20 ml of oxygen) | |

The hair dye composition prepared as mentioned above was packed into a tube in the same way as in Example 3. The tube was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in gray black. This color was the same as that obtained with the fresh composition. The hair treated with this hair dye composition gave a good touch due to treatment effect.

### [Example 17] (Hair dye composition of one-pack type, in gel form)

| | |
|---|---|
| p-Phenylenediamine | 1.0 % |
| 2,5-Diaminotoluene sulfate | 2.0 |
| m-Phenylenediamine | 0.5 |
| p-Aminophenol | 0.5 |
| 2-(2'-Hydroxyethylamino)-5-aminotoluene sulfate | 0.15 |
| Oleic acid | 0.2 |
| β-Cyclodextrin | 1.0 |
| Laccase | 1.0 |
| Xanthan gum | 0.5 |
| Hydroxyethylcellulose | 1.0 |
| POE (40) lauryl ether | 1.0 |
| POE (30) hydrogenated castor oil | 1.0 |
| Ethanol | 10.0 |
| Glycolic acid | 0.2 |
| Purified water | Balance |
| Total | 100.0% |
| pH : 7.5 (adjusted with monoethanolamine) | |
| Provided with oxygen absorber (capable of absorbing 20 ml of oxygen) | |

The hair dye composition prepared as mentioned above was packed into a tube in the same way as in Example 2. The tube was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in blue black. This color was the same as that obtained with the fresh composition.

### [Example 18] (Hair dye composition of one-pack type, in gel form)

| | |
|---|---|
| 5,6-Dihydroxyindoline hydrobromide | 1.0 % |
| 5,6-Dihydroxyindole hydrochloride | 1.0 |
| N-Ethyl-5,6-dihydroxyindole hydrochloride | 0.05 |
| Oleic acid | 0.2 |
| β-Cyclodextrin | 1.0 |
| Laccase | 5.0 |
| Xanthan gum | 0.5 |
| Hydroxyethylcellulose | 1.0 |
| POE (40) lauryl ether | 1.0 |
| POE (30) hydrogenated castor oil | 1.0 |
| Ethanol | 10.0 |
| Glycolic acid | 0.2 |
| Purified water | Balance |
| Total | 100.0% |
| pH : 7.5 (adjusted with monoethanolamine) | |
| Provided with oxygen absorber (capable of absorbing 20 ml of oxygen) | |

The hair dye composition prepared as mentioned above was packed into a tube in the same way as in Example 3. The tube was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in black. This color was the same as that obtained with the fresh composition.

As mentioned above, the present invention gives a hair dye composition of one-pack type which produces a good hair-dyeing effect and remains stable for a long time, with laccase reacting very little with the dye precursor to form aggregates.

## Claims

1. A laccase-containing oxidation hair dye composition of one-pack type in the form of cream or gel which is packed into a container with a good oxygen barrier property.

2. A laccase-containing oxidation hair dye composition of one-pack type which is packed into a container having an oxygen absorber in its head space.
